# EUROPÄISCHE PATENTSCHRIFT

(11) **EP 1 905 464 B1**
(45) Veröffentlichungstag und Bekanntmachung des Hinweises auf die Patenterteilung: **16.05.2012**
(21) Anmeldenummer: 07017118.6
(22) Anmeldetag: 31.08.2007
(51) Int. Cl.: A61L 27/48, A61L 27/50

(54) **Implantat und Verfahren zu seiner Herstellung**
Implant and method for its production
Implant et son procédé de fabrication

(30) Priorität: 05.09.2006 DE 102006042631
(43) Veröffentlichungstag der Anmeldung: 02.04.2008
(73) Patentinhaber: JOTEC GmbH, 72379 Hechingen (DE)
(72) Erfinder: Fischer, Heike, Dr., 40670 Meerbusch (DE); Schulze, Monika, 72379 Hechingen (DE)
(74) Vertreter: Laufer, Gabriele

(56) Entgegenhaltungen:
- EP-A1- 0 562 864
- EP-A1- 1 275 405
- DE-A1- 3 203 957
- US-A1- 2004 110 439
- THOMAS SCHEIBEL: "Protein fibers as performance proteins: new technologies and applications" CURRENT OPINION IN BIOTECHNOLOGY, Bd. 16, 2005, Seiten 427-433, XP002597847

## Beschreibung

Die Erfindung betrifft ein Implantat, insbesondere eine Gefäßprothese, welche eine strukturierte Materialkomponente und eine Proteinmatrix mit Porenstruktur aufweist, sowie Verfahren zu seiner Herstellung und seine Verwendung.

Implantate und Verfahren zu ihrer Herstellung sind aus dem Stand der Technik vielfach bekannt. Unter "Implantaten" werden im Allgemeinen Vorrichtungen verstanden, die in den Körper eines Patienten zumindest vorübergehend eingesetzt werden, und die beispielsweise therapeutische, Stütz- oder Gelenkfunktionen ausüben sollen.

Vor allem im Gebiet des Tissue Engineerings finden implantierbare Strukturen ihre Anwendung, einem interdisziplinären Forschungsgebiet, das sich mit Verfahren und Materialien zur Herstellung von künstlichen Gewebe- und Organsystemen befasst. So können beispielsweise künstlich hergestellte Implantate als Haut-, Knochen-, Knorpel-, Linsen- oder Gefäßersatz Anwendung finden.

Implantate in Form von Gefäßprothesen werden als Ersatz eines natürlichen erkrankten Gefäßes eingesetzt. Der erkrankte Gefäßabschnitt wird entfernt und durch ein Implantat ersetzt. So werden in der Gefäßchirurgie beispielsweise kleinlumige Implantate vor allem dann eingesetzt, wenn die körpereigenen Gefäße eines Patienten nicht verwendbar sind. Dies ist z.B. der Fall, wenn eine spezifische Gefäßlänge benötigt wird, oder wenn die autologen Gefäße auf Grund pathophysiologischer Eigenschaften nicht einsetzbar sind. Hier kommen Gefäßprothesen aus Kunststoff zum Einsatz, wobei vor allem Kunststoffe wie z.B. gewirkte oder gewebte Fäden aus Polyethylenterephthalat (PET) oder Gefäßprothesen aus expandiertem Polytetrafluorethylen (ePET) verwendet werden.

Gefäßimplantate aus diesen Kunststoffen werden bevorzugt verwendet, da sie vorteilhafte strukturelle und biokompatible Eigenschaften besitzen. So kann einerseits umliegendes Gewebe einwachsen, andererseits darf durch die Poren kein Blutplasma austreten. Dies wird bei den ePET-Implantaten durch die eingestellte Größe der Poren erzielt, während gewirkte und gewebte PET-Implantate durch eine Beschichtung mit resorbierbaren Materialien wie beispielsweise Kollagen oder Gelatine imprägniert werden. Nach Implantation wird die Beschichtung in dem Maße resorbiert, in dem das umliegende neu gebildete Gewebe in die poröse Kollagenschicht einwächst.

Es ist bekannt, dass der Einsatz der oben beschriebenen Implantate im kleinlumigen Gefäßbereich zu hohen Verschlussraten führt. Denn insbesondere der Kontakt von langsam strömendem Blut mit künstlichen Oberflächen kann zur Aktivierung des Gerinnungssystems, des Komplementsystems und des Immunsystems führen.

Weitergehende Ansätze zur Vermeidung von Blutgerinnung gehen dahin, die beschichteten Implantate mit Zellen, wie z.B. Endothelzellen und Zellen der glatten Muskulatur, zu besiedeln. Die Interaktion der verschiedenen Zellarten, die beispielsweise in natürlichen Gefäßen vorhanden sind, sowie die Zell-Matrix-Interaktion sind für die Funktionalität der Implantate bedeutend. So muss neben einer hohen Biokompatibilität auch gewährleistet sein, dass die Struktur des Implantats an die Anforderungen von verschiedenen Zellen angepasst ist.

An Implantate werden besondere mechanische und strukturelle Anforderungen gestellt. So sollen sie neben einer ausreichenden Strukturstabilität auch ein auf das zu ersetzende Gewebe abgestimmtes Kraft- und Dehnungsverhalten besitzen. Implantate müssen außerdem verschiedene Passformen, Längen und Durchmesser besitzen. Zudem spielt die Mikrostrukturierung, wie z.B. beim bioartifiziellen Gefäßersatz die radial verlaufende Porenstruktur mit zellspezifischer Größe, eine wichtige Rolle für die Besiedelung mit Zellen und für das wachsende Gewebe.

Für die Bereitstellung einer geeigneten Porenstruktur werden im Stand der Technik u.a. Schwämme, bzw. einen Schwamm aufweisende Implantate bereitgestellt. So ist beispielsweise aus der WO 99/27315 ein Verfahren zur Herstellung poröser Strukturen bekannt, bei welchem eine flüssige oder pastöse Mischung durch zweiseitiges Abkühlen zur Erstarrung gebracht wird. Auf diese Weise kann eine homogene poröse Struktur erzeugt werden.

Aus der EP 0 562 864 sind ferner heteromorphe Schwämme bekannt, die aktive Inhaltsstoffe aufweisen. Die heteromorphen Schwämme weisen darüber hinaus zumindest eine Substruktur auf, die wie die Matrixstruktur des Schwammes aus absorbierbaren Biopolymer-Materialien hergestellt sind. Der Beschreibung dieser Patentanmeldung ist zu entnehmen, dass bei der Herstellung der heteromorphen Schwämme zunächst ein entsprechender Schwamm durch Einfrieren hergestellt wird, und anschließend wird ein Kollagen-Film auf den eingefrorenen Schwamm gelegt, eine weitere Schicht Kollagen-Suspension über den Kollagen-Film aufgebracht und dieses Komposit anschließend gefriergetrocknet.

Nachteilig an diesem Implantat ist jedoch, dass keine ausgerichtete Porenstruktur des Schwammes erzielt werden kann, was darin begründet ist, dass mittels eines Gebläses eingefroren wird. Ferner wird durch das Aufliegen der Kollagen-Folie und weiteres Auftragen einer Kollagen-Suspension und deren Einfrieren keine Verankerung des Kollagen-Films in der schwammartigen Struktur erreicht.

Eine Verankerung der verschiedenen Schichten ist jedoch erwünscht, um dem Implantat eine hinreichende Stabilität und Elastizität zu verleihen, um das Implantat dementsprechend flexibel handhaben zu können.

Aus der DE 101 35 275 ist ein Implantat sowie ein Verfahren zu seiner Herstellung bekannt, das eine Proteinmatrix mit einer gerichteten Porenstruktur aufweist, wobei die Proteinmatrix in einer weiteren Schicht (Fremdstruktur) verankert ist. Die in der DE 101 35 275 offenbarten Implantate haben gegenüber den zuvor beschriebenen Implantaten den Vorteil, dass sie aufgrund des Herstellungsverfahrens eine gerichtete Porenstruktur aufweisen, aufgrund dessen beispielsweise Zellen optimal einwachsen können.

Nachteilig an dem aus der DE 101 35 275 bekannten Implantats ist, dass das unbesiedelte Implantat direkt nach Zellaufgabe nicht dicht ist, wodurch es während der Besiedelung unter Strömung zu einem Medienaustritt am Gefäßeingang und einem Medieneintritt am Ende des durchströmten Gefäßes kommen kann. Die Ausbildung eines Endothelmonolayers ist daher nur schwer möglich.

EP0562864 offenbart ein Implantat, mit einer strukturierten Materialkomponente und mit einer Proteinmatrix, die eine Porenstruktur aufweist.

DE3203957 und US2004/110439 offenbaren ein Implantat mit poröser Proteinmatrix und ein Verfahren zu seiner Herstellung.

T. Scheibel in Current Opinion in Biotechnology, 2005,16:427-433, offenbart eine poröse Proteinmatrix und ein Verfahren zu deren Herstellung.

Obwohl mit den gegenwärtig auf dem Markt vertriebenen Implantaten ein breites Spektrum an Implantaten zur Verfügung steht, besteht dennoch das große Bedürfnis, weitere Ausführungsformen bereitzustellen, bei welchen die für Implantate essentiellen Eigenschaften wie hinreichende Stabilität bei gleichzeitiger Flexibilität verbessert sind, welche eine gute Handhabung ermöglichen und ferner biokompatibel sowie gut resorbierbar sind.

Der Erfindung liegt daher die Aufgabe zugrunde, ein Implantat sowie ein Verfahren zu seiner Herstellung bereitzustellen, mit welchem zum einen die Nachteile der im Stand der Technik offenbarten Implantate überwunden werden können und welches die eben beschriebenen Vorteile erfüllen kann.

Bei dem eingangs genannten Implantat wird die Aufgabe erfindungsgemäß durch ein Implantat gelöst, bei welchem die strukturierte Materialkomponente zumindest teilweise in der Proteinmatrix verankert ist und bei welchem das Implantat auf zumindest einer Oberfläche eine Proteinmembran aufweist.

Ferner wird die Aufgabe gelöst durch ein Verfahren zur Herstellung eines Implantats, das die erfindungsgemäße Struktur aufweist.

Die der Erfindung zugrunde liegende Aufgabe wird hiermit vollständig gelöst.

Mit der auf dem Implantat vorgesehenen Proteinmembran wird eine Oberfläche bereitgestellt, die für die Besiedelung mit Zellen, beispielsweise Endothelzellen, und für die zügige Ausbildung eines Zellmonolayers geeignet ist. Die Proteinmembran ist derart ausgebildet, dass sie flüssigkeitsdicht ist, ohne eine Diffusionsbarriere darzustellen, und damit undurchlässig für einen Strömungsaus- und -eintritt ist.

Im Falle der Ausbildung des Implantats als Gefäßimplantat bildet die Proteinmembran die Gefäßinnenseite, wodurch diese Gefäßinnenseite zur Ausbildung eines Endothelmonolayers mit Endothelzellen besiedelt wird.

Im Falle einer Ausbildung des Implantats als Gewebeimplantat ist die Proteinmembran auf der Oberfläche als abgrenzende Schicht angeordnet.

Unter "strukturierte Materialkomponente" wird dabei jede fortlaufende, bzw. zusammenhängende Struktur verstanden, die schichtartig ausgebildet ist und eine Art Gerüst für das Implantat bildet, welches entweder in der Proteinmatrix gänzlich enthalten ist, oder aber als Begrenzungsschicht dient, in welche die Proteinmatrix teilweise eingebracht sein kann. "Strukturiert" bedeutet hierbei, dass die Materialkomponente Poren oder Hinterschneidungen aufweist, über welche die Materialkomponente in der Proteinmatrix verankert werden kann.

Durch das erfindungsgemäße Implantat können erstmals Gefäß- und/oder Gewebeimplantate bereitgestellt werden, die eine hinreichende Stabilität aufweisen, die - je nach Wahl der strukturierten Materialkomponente - vollständig oder nahezu vollständig resorbieren und die durch ihre Mikrostrukturierung an die spezifischen Zellanforderungen während der Zellaufgabe, der Kultivierung und der Gewebeneubildung angepasst sind. So bildet die Proteinmembran eine geeignete Grundlage für das Anheften von Zellen und die Ausbildung eines Zellmonolayers, wie beispielsweise die Ausbildung eines Endothelmonolayers beim bioartifiziellen Gefäßimplantat. Die Proteinmatrix mit gerichtet (d.h. in eine einzige Richtung) verlaufender, offener Porenstruktur mit angepasster Porengröße ermöglicht während der Besiedelung eine gleichmäßige Zellverteilung innerhalb der Matrix. Die Zellen sind ähnlich der nativen Situation in eine Proteinmatrix eingebettet. Beim bioartifiziellen Gefäßersatz wir die Porenstruktur beispielsweise an die Anforderungen der glatten Muskelzellen angepasst.

In einer Ausführungsform ist dabei bevorzugt, wenn die strukturierte Materialkomponente vollständig in der Proteinmatrix verankert ist.

Bei dieser Ausführungsform durchwachsen die in der Proteinmatrix vorliegenden Poren die strukturierte Materialkomponente, so dass die strukturierte Materialkomponente vollständig von der Proteinmatrix umgeben ist, bzw. in der Proteinmatrix aufgenommen ist. Die Poren oder Maschen der strukturierten Materialkomponente sind um ein Vielfaches größer als die der Proteinmatrix und bilden somit keine Zellbarriere. Gleichzeitig wird durch die in der Proteinmatrix eingelagerte strukturierte Materialkomponente der schwammartigen Struktur der Proteinmatrix eine hinreichende Stabilität verliehen. Ferner wird durch die auf der Oberfläche angebrachte Proteinmembran gleichzeitig eine flüssigkeitsdichte Struktur als Grenze zur Umgebung bereitgestellt, also eine Art Abdichtung geschaffen, die beispielsweise bei Ausarbeitung des Implantats als Gefäßimplantat als Blutaustrittsbarriere dient.

Durch die Einbringung der Proteinmembran ist das Implantat bereits bei Zellaufgabe flüssigkeitsdicht. Dadurch kann vorteilhafterweise die Besiedelung unter definierten Scherraten durchgeführt werden. Die Besiedelungszeit bis zur Ausbildung eines Zellmonolayers kann dadurch vorteilhaft verkürzt werden.

In einer weiteren Ausführungsform ist bevorzugt, wenn die strukturierte Materialkomponente nur teilweise in die Proteinmatrix eingebracht ist und die strukturierte Materialkomponente eine äußere Begrenzungsschicht des Implantats bildet.

Diese Ausführungsform des Implantats weist somit einen schichtartigen Aufbau auf, wobei die Schicht der Proteinmatrix zwischen einerseits der strukturierten Materialkomponente, in welche sie teilweise verankert ist, und der Proteinmembran andererseits eingebracht ist.

Die strukturierte Materialkomponente kann dabei jedes Material aufweisen, das eine strukturierte Form bildet bzw. vorgibt. Als strukturierte Materialkomponente geeignet sind daher bspw. strukturierte beständige künstliche Materialien, strukturierte resorbierbare künstliche Materialien, strukturierte natürliche Materialien, sowie Mischungen aus den genannten Materialien.

Ferner ist bevorzugt, wenn die strukturierte Materialkomponente ein Material aufweist, das ausgewählt ist aus der Gruppe umfassend Polytetrafluorethylen, Polyurethan, Polystyrol, Polyester, Keramik, Metall, Polylactit, Poly-Glykolsäure, Polyhydroxyalkanoate, Copolymere davon, Polysaccharide oder Mischungen aus einem oder mehrerer der genannten Materialien.

In einer bevorzugten Ausführungsform weist die strukturierte Materialkomponente Polyester auf, welches im Falle einer Gefäßprothese als großmaschiger netzartiger Schlauch ausgebildet sein kann. Gleichwohl sind auch andere Materialien als schlauchartige Grundgerüste der Fremdstruktur denkbar, bspw. expandiertes Polytetrafluorethylen, das sich in den letzten Jahren als bevorzugtes künstliches Material für Implantate durchgesetzt hat.

Es ist ferner bevorzugt, wenn die Proteinmembran ein Material aufweist, das ausgewählt ist aus der Gruppe umfassend Kollagen, Elastin, Cellulose, Chitosan, Chitin und Komponenten der Extrazellulären Matrix oder eine Mischung zweier oder mehrerer dieser Stoffe.

Die Proteinmembran kann dabei in einer bevorzugten Ausführungsform eine Schichtdicke von zwischen 0,02 mm und 5 mm aufweisen.

Bei dem erfindungsgemäßen Implantat ist ferner bevorzugt, wenn die Proteinmatrix eine Porengröße von ca. 5 µm bis 500 µm aufweist.

Ferner ist bevorzugt, wenn die Proteinmatrix, in welche die strukturierte Materialkomponente eingebracht ist, eine Schichtdicke von ca. 0,05 mm bis ca. 50 mm aufweist.

In einer bevorzugten Ausführungsform weist das Implantat die Ausbildung einer röhrenförmigen Gefäßprothese/Gefäßimplantats auf, wobei bevorzugt ist, wenn die Proteinmembran in der Innenseite der röhrenförmigen Gefäßprothese ausgebildet ist.

Mit dieser Ausführungsform wird demnach eine Gefäßprothese bereitgestellt, die auf Grund ihrer Ausbildung einerseits die Regeneration der Gefäßwand, eine Neovaskularisierung aus dem umliegenden Gewebe und eine gute Nährstoffversorgung in der Proteinmatrix gewährleistet, und die andererseits auf Grund der Innenauskleidung mit der Proteinmembran ein Austreten von Blut in das umgebende Gewebe verhindert. Gleichzeitig wird mit der Proteinmembran eine Schicht für die Besiedelung mit Endothelzellen bereitgestellt, über welche ein Endothelmonolayer einfach ausgebildet werden kann.

In weiteren Ausführungsformen ist bevorzugt, wenn das Implantat ferner zumindest einen Wirkstoff aufweist, der ausgewählt ist aus der Gruppe umfassend Heparin, Hirudin, Aspirin, Heparansulfat, Albumin, Antibiotika, wie beispielsweise Rifampicin, und/oder Wachstumsfaktoren.

Je nach Anwendung können die Implantate vor der Implantation mit Wirkstoffen beladen oder beschichtet werden. Dabei kann die Wirkstoffabgabe anschließend beispielsweise durch eine zusätzlich aufgebrachte Hydrogelbeschichtung oder durch die Art der Anbindung der Wirkstoffe noch zusätzlich kontrolliert werden.

Dabei ist in einer Ausführungsform bevorzugt, wenn die Proteinmembran ohne Vernetzungsmittel mit der Proteinmatrix vernetzt ist.

Dies wird bspw. dadurch erreicht, dass die Proteinmembran durch einen Abkühlvorgang bei der Herstellung des Implantats mit der Proteinmatrix vernetzt wird.

In einer anderen Ausführungsform ist bevorzugt, wenn die Vernetzung der Proteinmembran mit der Proteinmatrix (oder eine weitere/zusätzliche Vernetzung) durch chemische Vernetzungsmittel (wie bspw. Glutaraldehyd) erreicht wird.

Die Erfindung betrifft ferner ein Verfahren zur Herstellung der erfindungsgemäßen Implantate, das die folgenden Schritte aufweist:
a) Bereitstellen einer Proteinmembran auf einer der Grundform der Proteinmembran entsprechenden ersten abgrenzenden Form in einer Anordnung;
b) Positionieren der strukturierten Materialkomponente über der Proteinmembran in der Anordnung, wobei die Form der Fremdstruktur der Grundform der Proteinmembran entspricht;
c) Aufbringen einer zweiten abgrenzenden Form auf die strukturierte Materialkomponente;
d) Einbringen einer Suspension zwischen Proteinmembran und strukturierter Materialkomponente, und
e) Abkühlen der Anordnung und anschließende Gefriertrocknung zur Ausbildung einer Proteinmatrix aus der Suspension und zur Anbindung der Proteinmembran.

Bei diesem Verfahren wird also zunächst eine vorgefertigte Proteinmembran auf eine - der Form der vorgefertigten Proteinmembran entsprechende - erste Form aufgebracht, wobei diese Form bei der Herstellung des Implantats eine erste Abgrenzung für das Implantat-Konstrukt bildet. Die Form kann ggf. während eines einseitigen Einfriervorgangs als Kühlplatte oder Isolation dienen. Anschließend wird eine strukturierte Materialkomponente über die Proteinmembran positioniert, wobei auf die strukturierte Materialkomponente eine zweite abgrenzende Form aufgebracht wird, die ggf. je nach Anordnung als Kühlplatte oder zur Isolation dienen kann. In einem nächsten Schritt wird eine Suspension zwischen die Proteinmembran und die strukturierte Materialkomponente eingebracht und die gesamte Anordnung abgekühlt und gefriergetrocknet. Vor der Gefriertrocknung wird eine der begrenzenden Formen entfernt, um den Gefriertrocknungsvorgang zu beschleunigen. Durch das Abkühlen bildet sich aus der Suspension eine Proteinmatrix aus, wobei gleichzeitig bei diesem Vorgang diese Proteinmatrix einerseits teilweise in der strukturierten Materialkomponente verankert und andererseits mit der Proteinmembran vernetzt wird.

Je nach Abstand der zweiten abgrenzenden Form von der Fremdstruktur können bspw. die in der Proteinmatrix entstehenden Poren bspw. durch die strukturierte Materialkomponente hindurch wachsen. In diesem Fall kann bspw. die durch die strukturierte Materialkomponente hindurch gewachsene Proteinmatrix nach Fertigstellung des Implantats dem Verwendungszweck entsprechend abgeschnitten bzw. gekürzt werden. Andererseits kann auch durch direktes Anlegen der abgrenzenden zweiten Form erreicht werden, dass die Poren nicht komplett durch die strukturierte Materialkomponente wachsen, sondern lediglich in der strukturierten Materialkomponente verankert werden.

In einer Ausführungsform des erfindungsgemäßen Verfahrens ist bevorzugt, wenn das Abkühlen in Schritt e) einseitig erfolgt. Durch das einseitige Abkühlen kann eine gerichtete Porenstruktur ausgebildet werden, durch welche ein gleichmäßiges Einbetten von Zellen während der Besiedelung ermöglicht wird. Bei diesem Verfahren wird die Anordnung auf einer Seite bzw. Oberfläche einseitig abgekühlt, während die andere Oberfläche isoliert wird. Während des Einfrierprozess kann die Suspension teilweise oder vollständig auskristallisieren.

Implantate, die nach dem bevorzugten Verfahren hergestellt werden, können beispielsweise - bei Verwendung eines Metallrohrs als Grundform - Gefäßimplantate darstellen, wobei jedoch auch flächige Gewebeimplantate unter Verwendung entsprechend flächiger Grundformen hergestellt werden können.

Bei der o.g. Ausführungsform des erfindungsgemäßen Verfahrens ist bevorzugt, wenn das Abkühlen in Schritt e) mit einer konstanten Kühlrate verläuft und insbesondere mit einer Kühlrate von ca. 0,1 K/min bis 200 K/min.

Durch Anwendung unterschiedlicher Kühlraten können verschiedene Porengrößen der Proteinmatrix eingestellt werden. Dabei gilt, dass die Porengröße umso kleiner ist, je schneller abgekühlt wird. Bspw. kann mit einer Kühlrate von 12 K/min eine Porengröße von ca. 45 µm bis 50 µm eingestellt werden und eine Porengröße von ca. 85 µm bis 105 µm bei einer Abkühlrate von 1 K/min, wobei die jeweiligen Bereiche bspw. durch die Zugabe von Säuren wie Essig- oder Ascorbinsäure beeinflusst werden können.

Die Anwendung des einseitigen Abkühlens hat sich bei der Herstellung des erfindungsgemäßen Implantats als besonders geeignet erwiesen, da auf diese Weise gerichtete Poren hergestellt werden können.

In einer anderen Ausführungsform des erfindungsgemäßen Verfahrens ist bevorzugt, wenn das Abkühlen in Schritt e) bei einer konstanten Temperatur erfolgt.

In dieser Variante kann die Anordnung bspw. in einer Gefriertruhe oder anderen geeigneten Kühl- bzw. Gefriervorrichtungen abgekühlt werden. Diese Implantate weisen jedoch dann keine gerichtete Porenstruktur auf, sind aber - je nach Anwendung - für einen Einsatz als Implantate ebenfalls geeignet.

In dem erfindungsgemäßen Verfahren ist ferner bevorzugt, wenn die Suspension, aus welcher die Proteinmatrix gebildet wird, eine Suspension ist, die Kollagen, Elastin und lösliche, nicht-kollagene Bestandteile enthält.

Zusätzliche Begleitstoffe können Wachstumsfaktoren oder Bestandteile der extrazellulären Matrix sein, wie z.B. Laminin, Elastin, Fibronektin, Hyaluronsäure, Glykosaminoglykane u.a., sowie Derivate davon. Die löslichen Bestandteile umfassen einerseits Säuren wie HCL, Essigsäure oder Ascorbinsäure, da bekannt ist, dass der optimale pH-Bereich zur Herstellung gefriergetrockneter Kollagenschwämme zwischen 2,5 und 3,5 liegt. Andererseits können lösliche Zusatzstoffe wie Glycerin oder Ethanol oder fein-dispergierte Stoffe wie bspw. Calciumphosphat eingesetzt werden, da durch ihre Konzentration die Eiskristallmorphologie und somit die Porenstruktur eingestellt werden kann.

Bei einer weiteren Ausführungsform ist vorgesehen, dass die Proteinmembran, die in Schritt a) eingesetzt wird, aus der gleichen Suspension hergestellt wird wie die Suspension zur Herstellung der Proteinmatrix, und -je nach Einsatz des Implantats - in planarer oder tubulärer Form ausgebildet wird.

Im Falle des einseitigen Einfrierprozess, während dessen die Suspension auskristallisieren kann, werden die Kollagene, Elastin und die gelösten Stoffe dabei von der wachsenden zellularen Einsphasenfront verschoben, wobei die suspensierten Proteine und die gelösten oder dispergierten Stoffe zwischen den Eiskristallen hoch aufkonzentriert werden. Durch die Kühlrate und die chemische Zusammensetzung der Proteinsuspension können die Eiskristallstruktur- und größe bei einer beliebigen Schichtdicke vorteilhaft eingestellt werden.

In einer Weiterbildung des erfindungsgemäßen Verfahren ist ein weiterer Schritt f) vorgesehen, mit welchem das in den Schritten a) bis e) hergestellte Implantat anschließend gefriergetrocknet wird.

Bei dem Gefriertrocknungsprozess sublimieren die Eiskristalle, wodurch Poren entstehen, die der Einkristallmorphologie der gefrorenen Probe entsprechen. Bei der Besiedelung der Proteinmatrix können Zellen gleichmäßig in den Poren verteilt werden. Des weiteren können bei einer Implantation die Zellen des umliegenden Gewebes entlang der Proteinfasern in das Implantat einwachsen. Durch den Wasserentzug während der Sublimation bilden sich kovalente Bindungen zwischen den Kollagenmolekülen aus, wodurch die Matrix, die Membran und die Verbindung Matrix-Membran eine erwünschte Stabilität erhält. Dabei ist es vorteilhaft, dass der Vernetzungsgrad durch den Gefriertrocknungsprozess oder durch eine chemische Behandlung des gefriergetrockneten Produkts gezielt eingestellt werden kann. Die durch den Gefriertrocknungsprozess entstandene Proteinmatrix ist somit direkt mit der Membran und der Fremdstruktur verankert und es sind keine weiteren Schritte zur Verbindung der Fremdstruktur und der Membran mit der Proteinmatrix notwendig.

Durch die Erfindung kann mit einfachen Mitteln erreicht werden, dass das Implantat den jeweiligen Anforderungen des umliegenden Gewebes durch die Ausbildung einer Porenstruktur und die Anbindung einer Membran einfach und effizient angepasst werden kann. Die so hergestellten Implantate weisen eine mechanische Stabilität und biologische Kompatibilität auf.

Die Erfindung betrifft ferner Implantate, die nach dem erfindungsgemäßen Verfahren hergestellt werden, sowie insbesondere Gefäßprothesen/Gefäßimplantate. Derartige Gefäßprothesen können bspw. als kleinlumiger Gefäßersatz (Durchmesser < 6 mm), z.B. bei Herzkranzgefäßen oder Gefäßen der Peripherie eingesetzt werden, ferner als Dialyseshunt oder in der Pädiatrie als mitwachsende Gefäßimplantate.

Weitere Vorteile ergeben sich aus der Beschreibung und der beigefügten Zeichnungen.

Es versteht sich, dass die vorstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Ausführungsbeispiele der Erfindung sind in den nachstehenden Zeichnungen dargestellt und werden in der nachfolgenden Beschreibung näher erläutert. Es zeigen:
- Fig. 1: einen Querschnitt eines Ausführungsbeispiels eines erfindungsgemäßen Implantats, nämlich einer Gefäßprothese;
- Fig. 2: ein Diagramm, das die Abhängigkeit der Porengröße von der Essigsäurekonzentration darstellt;

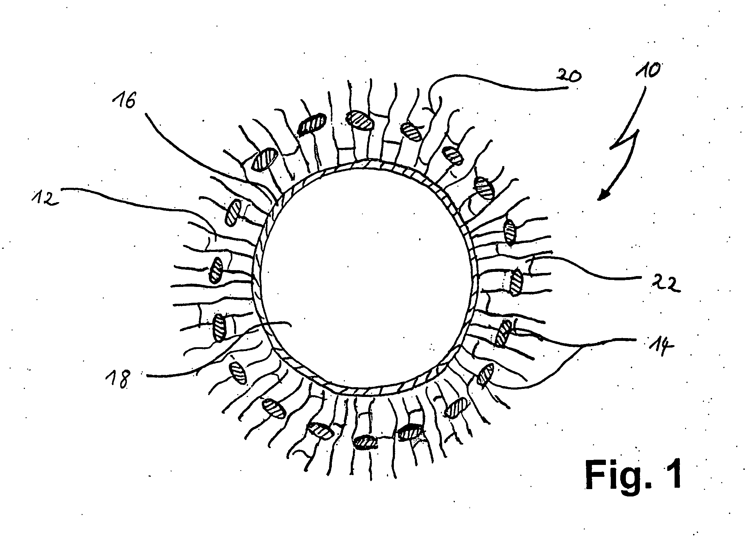

In Fig. 1 bezeichnet 10 insgesamt ein Gefäßimplantat 10, das nach dem erfindungsgemäßen Verfahren hergestellt wurde. Bei dem Implantat ist eine Proteinmatrix 12 in einer strukturierten Materialkomponente 14 tief verankert. Die Innenwand des Gefäßimplantats 10 wird durch eine Proteinmembran 16 gebildet, welche die Grenze zum Lumen 18 darstellt. Die Proteinmatrix 12 besteht aus Proteinfasern 20 mit Poren 22.

Durch die strukturierte Materialkomponente 14, die gerüstartig in der Proteinmatrix vorliegt, wird dem Gefäßimplantat 10 eine ausreichende Stabilität und Nähfestigkeit verliehen. Die gerichtet verlaufenden Poren 22 der Proteinmatrix 12 können gleichmäßig mit Zellen, wie beispielsweise glatten Muskelzellen, besiedelt werden, bzw. *in vivo* können Zellen aus dem umgebenden Gewebe einwachsen. Andererseits kann die Innenwand, bzw. Proteinmembran 16, des Gefäßimplantats 10 mit Endothelzellen besiedelt werden, da durch die Proteinmembran 16 eine geeignete Oberfläche zur Ausbildung eines Endothelmonolayer bereitgestellt wird.

An dieser Stelle wird angemerkt, dass das in Fig. 1 gezeigte Gefäßimplantat 10 nur ein Ausführungsbeispiel verkörpert. Die Erfindung ist daneben auch in anderen Formen und Funktionen einsetzbar wie beispielsweise als Patches bei Hautimplantaten, als Zylinder oder Rechteck bei Knorpel- und Knochenimplantaten oder Herzklappen.

Durch das Verfahren können Implantate mit unterschiedlichen Schichtdicken der Proteinmatrix hergestellt werden. Um eine bestimmte Proteinmatrixschichtdicke zu erreichen, wird ein bestimmter Temperaturverlauf angelegt.

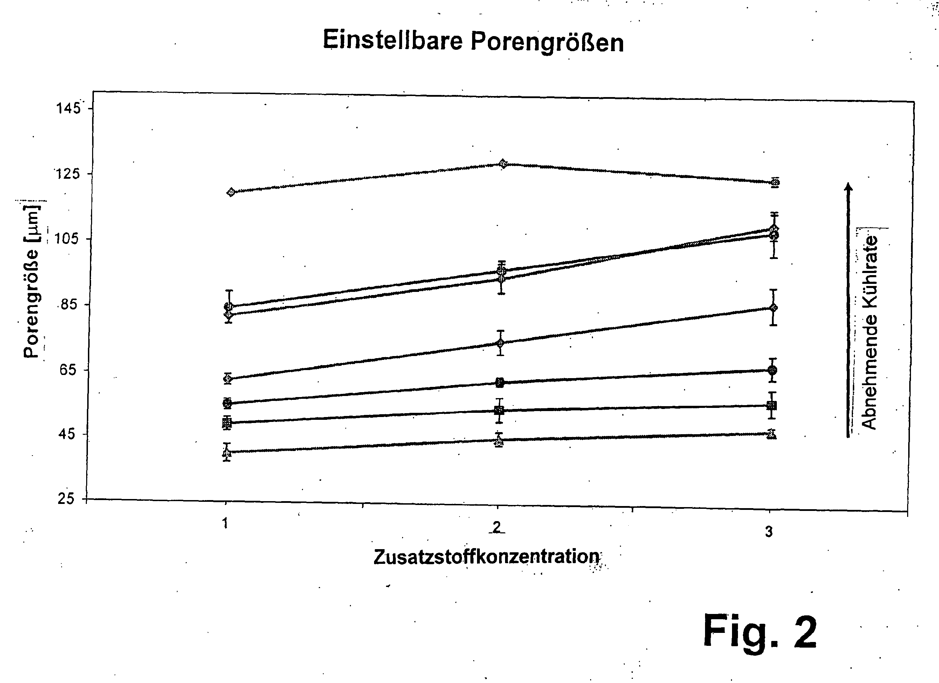

In diesem Zusammenhang ist in Fig. 2 ein Diagramm gezeigt, in welchem die Abhängigkeit der Porengröße unter Berücksichtigung der Essigsäurekonzentration und der Kühlrate dargestellt ist. Dem Diagramm ist zu entnehmen, dass bei einer geringen Abkühlrate größere Poren hergestellt werden können (s. beispielsweise 0,5 K/min im Vergleich zu 12 K/min) und dass bei gleichzeitiger Erhöhung der Essigsäurekonzentration die Porengröße ebenfalls größer war als bei einer geringeren Essigsäurekonzentration (s. beispielsweise bei der Abkühlrate von 1 K/min: 1,5 % Essigsäure: 85 µm Porengröße im Vergleich zu 3,8 % Essigsäure: ca. 110 µm Porengröße).

An Hand des unten beschriebenen Beispiels wird nun die Anwendung der Vorrichtung gezeigt.

### Beispiel

### Herstellung eines erfindungsgemäßen Gefäßimplantats

Nach bekannten Verfahren wurde ein großmaschiges texturiertes Polyesternetz gewirkt, gereinigt und geschrumpft. In einem Exsikkator wurde das Polyesternetz in Kollagen-Suspension vollständig entlüftet.

Aus der Kollagen-Suspension oder der Kollagen-Elastin-Suspension wurde durch Extrusion oder durch Herstellen einer flächigen Kollagenmembran und anschließende Röhrenbildung mit Kollagennaht eine röhrenförmige Kollagen-/Kollagen-Elastin-Membran mit dem gewünschten Innendurchmesser des entstehenden Gefäßimplantates hergestellt. Diese Kollagen-/Kollagen-Elastin-Membran (Proteinmembran) wurde in gewünschter Länge auf bspw. einem Metallrohr angebracht; über die Proteinmembran wurde das Polyesternetz (also die strukturierte Materialkomponente) geschoben und eine zweite röhrenförmige Form auf die strukturierte Materialkomponente aufgebracht, die je nach Ausführung als Kühlplatte oder zur Isolation dienen kann. In einem nächsten Schritt wurde eine (Kollagen-/Kollagen-Elastin)Suspension zwischen Proteinmembran und strukturierte Materialkomponente eingefüllt.

Anschließend wurde die Anordnung abgekühlt, d.h. die Kollagen-/Kollagen-Elastin-Suspension wird - bspw. durch einen einseitig kontrollierten Einfriervorgang - gerichtet erstarrt. Dazu wird die Temperatur des Metallrohres, welches nun als Kühlrohr fungiert, mit einer konstanten Kühlrate von beispielsweise 6 K/min abgesenkt. Als Kühlmedium dient Ethanol, welches kontinuierlich durch das Metallrohr gepumpt wird.

Nach dem Einfrieren wird die zweite Form entfernt. Die Proben werden bei < -45°C mindestens 12 Stunden gelagert und anschließend gefriergetrocknet.

Nach einer darauffolgenden Sterilisation ist die nach dem erfindungsgemäßen Verfahren hergestellte Gefäßprothese aus einer Kollagen-/Kollagen-Elastin-Matrix, Kollagen-/Kollagen-Elastin-Membran und einer Polyesterverstärkung zur Besiedelung mit bspw. Myofibroblasten, Endothelzellen und Zellen der glatten Muskulatur in einem Zellreaktor bereit.

Wie bereits erwähnt, ist das Implantat durch die Einbringung der Proteinmembran bereits bei Zellaufgabe flüssigkeitsdicht. Mit dem erfindungsgemäßen Implantat konnte die Besiedelungszeit bis zur Ausbildung eines Zellmonolayers auf höchstens vier Tage reduziert werden.

## Patentansprüche

1. Implantat mit einer strukturierten Materialkomponente und mit einer Proteinmatrix, die eine Porenstruktur aufweist, **dadurch gekennzeichnet, dass** die strukturierte Materialkomponente zumindest teilweise in der Proteinmatrix verankert ist und dass das Implantat auf zumindest einer Oberfläche ferner eine mit der Proteinmatrix vernetzte Proteinmembran aufweist.

2. Implantat nach Anspruch 1, **dadurch gekennzeichnet, dass** die strukturierte Materialkomponente vollständig in der Proteinmatrix verankert ist.

3. Implantat nach Anspruch 1, **dadurch gekennzeichnet, dass** die strukturierte Materialkomponente nur teilweise in die Proteinmatrix eingebracht ist und die strukturierte Materialkomponente eine äußere Begrenzungsschicht des Implantats bildet.

4. Implantat nach einem der Ansprüche 1 bis 3, **dadurch gekennzeichnet, dass** die strukturierte Materialkomponente ein Material aufweist, das ausgewählt ist aus der Gruppe umfassend Polytetrafluorethylen, Polyurethan, Polystyrol, Polyester, Keramik, Metall, Poly-Lactid, Poly-Glykolsäure, Polyhydroxyalkanoate, Copolymere davon, Polysaccharide oder Mischungen aus einem oder mehrerer davon.

5. Implantat nach einem der Ansprüche 1 bis 4, **dadurch gekennzeichnet, dass** die Proteinmatrix eine Porengröße von ca. 5 µm bis 500 µm aufweist.

6. Implantat nach einem der Ansprüche 1 bis 5, **dadurch gekennzeichnet, dass** die Proteinmatrix, in welche die Fremdstruktur eingebracht ist, eine Schichtdicke von ca. 0,05 mm bis ca. 50 mm aufweist.

7. Implantat nach einem der Ansprüche 1 bis 6, **dadurch gekennzeichnet, dass** das Implantat als röhrenförmige Gefäßprothese ausgebildet ist.

8. Implantat nach Anspruch 7, **dadurch gekennzeichnet, dass** die Proteinmembran in der Innenseite der röhrenförmigen Gefäßprothese ausgebildet ist.

9. Implantat nach einem der Ansprüche 1 bis 8, **dadurch gekennzeichnet, dass** das Implantat ferner einen Wirkstoff aufweist, der ausgewählt ist aus der Gruppe umfassend Heparin, Hirudin, Aspirin, Heparansulfat, Albumin, Antibiotika, Wachstumsfaktoren, oder Mischungen aus einem oder mehrerer dieser Wirkstoffe.

10. Implantat nach einem der Ansprüche 1 bis 9, **dadurch gekennzeichnet, dass** die Proteinmembran ohne Vernetzungsmittel mit der Proteinmatrix vernetzt ist.

11. Implantat nach einem der Ansprüche 1 bis 9, **dadurch gekennzeichnet, dass** die Proteinmembran über Vernetzungsmittel mit der Proteinmatrix verbunden ist.

12. Implantat nach Anspruch 11, **dadurch gekennzeichnet, dass** das Vernetzungsmittel ausgewählt ist aus der Gruppe umfassend Glutaraldehyd, Formaldehyd, Isocyanate, Ethylendi-chlorid, oder Mischungen aus einem oder mehrerer dieser Vernetzungsmittel.

13. Implantat nach einem der Ansprüche 1 bis 13, **dadurch gekennzeichnet, dass** die Proteinmatrix eine gerichtete Porenstruktur aufweist.

## Claims

1. Implant having a structured material component and having a protein matrix which has a pore structure, **characterized in that** the structured material component is at least partly anchored in the protein matrix, and **in that** the implant has on at least one surface a protein membrane crosslinked with the protein matrix.

2. Implant according to Claim 1, **characterized in that** the structured material component is completely anchored in the protein matrix.

3. Implant according to Claim 1, **characterized in that** the structured material component is only partly introduced into the protein matrix, and the structured material component forms an outer boundary layer of the implant.

4. Implant according to any of claims 1 to 3, **characterized in that** the structured material component includes the material which is selected from the group comprising polytetrafluoroethylene, polyurethane, polystyrene, polyester, ceramic, metal, polylactide, polyglycolic acid, polyhydroxyalkanoates, copolymers thereof, polysaccharides or mixtures of one or more thereof.

5. Implant according to any of Claims 1 to 4, **characterized in that** the protein matrix has a pore size of about 5 µm to 500 µm.

6. Implant according to any of Claims 1 to 5, **characterized in that** the protein matrix into which the foreign structure is introduced has a layer thickness of about 0.05 mm to about 50 mm.

7. Implant according to any of Claims 1 to 6, **characterized in that** the implant is configured as a tubular vascular prosthesis.

8. Implant according to Claim 7, **characterized in that** the protein membrane is formed in the inner side of the tubular vascular prosthesis.

9. Implant according to any of Claims 1 to 8, **characterized in that** the implant further includes an active ingredient which is selected from the group comprising heparin, hirudin, aspirin, heparan sulphate, albumin, antibiotics, growth factors, or mixtures of one or more of these active ingredients.

10. Implant according to any of Claims 1 to 9, **characterized in that** the protein membrane is crosslinked with the protein matrix without crosslinker.

11. Implant according to any of Claims 1 to 9, **characterized in that** the protein membrane is connected to the protein matrix by crosslinker.

12. Implant according to Claim 11, **characterized in that** the crosslinker is selected from the group comprising glutaraldehyde, formaldehyde, isocyanates, ethylene dichloride, or mixtures of one or more of these crosslinkers.

13. Implant according to any of Claims 1 to 13, **characterized in that** the protein matrix has a directed pore structure.

## Revendications

1. Implant comportant un composant de matériau structuré et une matrice de protéine qui présente une structure poreuse, **caractérisé en ce que** le composant de matériau structuré est ancré au moins en partie dans la matrice de protéine et **en ce que** l'implant présente en outre sur au moins une surface, une membrane protéique réticulée avec la matrice de protéine.

2. Implant selon la revendication 1, **caractérisé en ce que** le composant de matériau structuré est complètement ancré dans la matrice de protéine.

3. Implant selon la revendication 1, **caractérisé en ce que** le composant de matériau structuré est intégré seulement partiellement dans la matrice de protéine et le composant de matériau structuré forme une autre couche de délimitation de l'implant.

4. Implant selon l'une des revendications 1 à 3, **caractérisé en ce que** le composant de matériau structuré présente un matériau qui est choisi dans le groupe comprenant le polytétrafluoroéthylène, le polyuréthane, le polystyrène, le polyester, la céramique, le métal, le polylactide, l'acide polyglycolique, le polyhydroxyalcanoate, des copolymères de ceux-ci, des polysaccharides ou des mélanges d'un ou plusieurs de ceux-ci.

5. Implant selon l'une des revendications 1 à 4, **caractérisé en ce que** la matrice de protéine présente une taille de pore d'environ 5 µm à 500 µm.

6. Implant selon l'une des revendications 1 à 5, **caractérisé en ce que** la matrice de protéine dans laquelle la structure étrangère est intégrée, présente une épaisseur de couche d'environ 0,05 mm à environ 50 mm.

7. Implant selon l'une des revendications 1 à 6, **caractérisé en ce que** l'implant est constitué sous forme de prothèse vasculaire tubulaire.

8. Implant selon la revendication 7, **caractérisé en ce que** la membrane protéique est formée à l'intérieur de la prothèse vasculaire tubulaire.

9. Implant selon l'une des revendications 1 à 8, **caractérisé en ce que** l'implant présente en outre une substance active qui est choisie dans le groupe comprenant l'héparine, l'hirudine, l'aspirine, le sulfate d'héparane, l'albumine, des antibiotiques, des facteurs de croissance ou des mélanges d'une ou plusieurs substances actives.

10. Implant selon l'une des revendications 1 à 9, **caractérisé en ce que** la membrane protéique est réticulée sans agent de réticulation avec la matrice de protéine.

11. Implant selon l'une des revendications 1 à 9, **caractérisé en ce que** la membrane protéique est liée par un agent de réticulation avec la matrice de protéine.

12. Implant selon la revendication 11, **caractérisé en ce que** l'agent de réticulation est choisi dans le groupe comprenant le glutaraldéhyde, le formaldéhyde, l'isocyanate, le dichlorure d'éthylène ou des mélanges d'un ou plusieurs de ces agents de réticulation.

13. Implant selon l'une des revendications 1 à 13, **caractérisé en ce que** la matrice de protéine présente une structure poreuse orientée.
